(19) Europäisches Patentamt
European Patent Office
Office européen des brevets

(11) **EP 1 874 364 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**03.01.2018   Patentblatt 2018/01**

(45) Hinweis auf die Patenterteilung:
**14.05.2014   Patentblatt 2014/20**

(21) Anmeldenummer: **06724498.8**

(22) Anmeldetag: **21.04.2006**

(51) Int Cl.:
*A61L 15/18* [(2006.01)]        *B01J 20/26* [(2006.01)]
*C08J 7/12* [(2006.01)]         *A61L 15/60* [(2006.01)]
*C08F 222/10* [(2006.01)]

(86) Internationale Anmeldenummer:
**PCT/EP2006/003694**

(87) Internationale Veröffentlichungsnummer:
**WO 2006/111402 (26.10.2006 Gazette 2006/43)**

(54) **OBERFLÄCHENNACHVERNETZTE SUPERABSORBER BEHANDELT MIT KIESELSÄURE-VERBINDUNG UND EINEM AL3+-SALZ**

SURFACE CROSS-LINKED SUPERABSORBER TREATED WITH A SILICA COMPOUND AND A AL3+ SALT

SUPER ABSORBANT REVETE A LA SURFACE ET TRAITE AVEC UN COMPOSE de SILICE ET UN SEL AL3+

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.04.2005   DE 102005018924**

(43) Veröffentlichungstag der Anmeldung:
**09.01.2008   Patentblatt 2008/02**

(73) Patentinhaber: **Evonik Degussa GmbH
45128 Essen (DE)**

(72) Erfinder:
• **FURNO, Franck
  47799 Krefeld (DE)**
• **SCHMIDT, Harald
  47918 Tönisvorst (DE)**
• **HERBE, Peter
  47228 Duisburg (DE)**
• **NIELINGER, Ursula
  47802 Krefeld (DE)**
• **KEUP, Michael
  45139 Essen (DE)**

(74) Vertreter: **Lang, Arne et al
Evonik Industries AG
Intellectual Property Management
Bau 1042A / PB 15
Paul-Baumann-Straße 1
45764 Marl (DE)**

(56) Entgegenhaltungen:
**EP-A- 1 358 892          WO-A-2004/037900
WO-A-2006/094907     WO-A-2006/111403
WO-A-2006/111404     WO-A1-2004/096304
WO-A2-2004/037903   WO-A2-2004/069915
DE-A1- 10 138 630      DE-A1- 10 249 822
DE-A1- 19 854 575      DE-A1- 19 909 653
US-A- 4 587 308          US-A- 5 684 106
US-A- 5 973 042          US-A1- 2003 125 684**

**EP 1 874 364 B2**

**Beschreibung**

Die vorliegende Erfindung betrifft ein Verfahren zur Herstellung oberflächen nachvernetzter wasserabsorbierender Polymergebilde, die durch dieses Verfahren erhältlichen wasserabsorbierende Polymergebilde, einen Verbund, ein Verfahren zur Herstellung eines Verbunds, chemische Produkte beinhaltend wasserabsorbierende Polymergebilde oder einen Verbund sowie die Verwendung wasserabsorbierender Polymergebilde oder des Verbunds in chemische Produkten.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikeln wie beispielsweise Babywindeln, Inkontinenzprodukten oder Damenbinden.

Bei den gegenwärtig kommerziell verfügbaren Superabsorbern handelt es sich im wesentlichen um vernetzte Polyacrylsäuren oder vernetzte Stärke-Acrylsäure-Pfropfpolymere, bei denen die Carboxylgruppen teilweise mit Natronlauge oder Kalilauge neutralisiert sind.

Aus ästhetischen Gründen und aus Umweltaspekten besteht zunehmend die Tendenz, die Sanitärartikel immer kleiner und dünner zu gestalten. Um ein gleichbleibendes Gesamtretentionsvermögen der Sanitärartikel zu gewährleisten, kann dieser Anforderung nur durch Reduktion des Anteils an großvolumigen Fluff entsprochen werden. Hierdurch fallen dem Superabsorber weitere Aufgaben hinsichtlich dem Transport und der Verteilung von Flüssigkeit zu, die sich als Permeabilitätseigenschaften zusammenfassen lassen.

Unter Permeabilität versteht man bei Superabsorbermaterialien die Fähigkeit, im gequollenen Zustand eindringende Flüssigkeiten zu transportieren und gleichmäßig innerhalb des gequollenen Gels zu verteilen. Dieser Prozess läuft über kapillaren Transport durch Zwischenräume zwischen den Gelpartikeln ab. Ein Flüssigkeitstransport durch gequollene Superabsorberpartikel selbst folgt den Gesetzen der Diffusion und ist ein sehr langsamer Prozess, der in der Nutzungssituation des Sanitärartikels keine Rolle bei der Verteilung der Flüssigkeit spielt. Bei Superabsorbermaterialien, die einen kapillaren Transport aufgrund mangelnder Gelstabilität nicht bewerkstelligen können, wurde durch Einbetten dieser Materialien in eine Fasermatrix eine Separation der Partikel voneinander unter Vermeidung des Gel-Blocking-Phänomens sichergestellt..In Windelkonstruktionen neuer Generation befindet sich in der Absorberschicht nur wenig oder überhaupt kein Fasermaterial zur Unterstützung des Flüssigkeitstransports. Die hier verwendeten Superabsorber müssen demnach eine ausreichend hohe Stabilität im gequollenen Zustand besitzen, damit das gequollene Gel noch eine ausreichende Menge an kapillaren Räumen besitzt, durch die Flüssigkeit transportiert werden kann.

Um Superabsorbermaterialien mit hoher Gelstabilität zu erhalten, kann einerseits der Grad der Vernetzung des Polymers erhöht werden, was zwangsläufig eine Verminderung der Quellfähigkeit und des Retentionsvermögens zur Folge hat.

Weiterhin können Methoden zur Nachbehandlung der Oberfläche von Polymerpartikeln zur Verbesserung der Superabsorbereigenschaften zum Einsatz kommen. Als Oberflächenbehandlung sind beispielsweise die Nachvernetzung des wasserabsorbierenden Polymergebildes an der Oberfläche, das in Kontakt bringen der Oberfläche mit anorganischen Verbindungen oder aber die Nachvernetzung der Oberfläche in Gegenwart anorganischer Verbindungen aus dem Stand der Technik bekannt.

DE-A-100 16 041 erzielt eine Wiederherstellung der durch mechanische Einwirkung geschädigten Gelpermeabilität wasserabsorbierender Polymerisate dadurch, dass ein solches Polymerisat nach einer Nachvernetzung mit einer Lösung von wenigstens einem Salz eines mindestens dreiwertigen Kations nachbehandelt wird.

WO-A-98/48857 offenbart superabsorbierende Polymere aufweisend eine verbesserte *Gel Bed Resiliency,* die durch trockenes Vermischen des Polymeren mit einem mehrwertigen Metallsalz und anschließendes in Kontakt bringen des Gemisches mit einem Bindemittel erhalten wurden. Eine derartige Abmischung mit anorganischen, feinteiligen Substanzen bringt Nachteile, wie etwa Entmischung oder Stauben, mit sich.

WO-A-98/49221 beschreibt das Wiederbefeuchten wasserabsorbierender Polymere mit einer wässrigen Lösung eines ein ein- oder mehrwertiges Metallsalz enthaltenden Additivs nach einer Hitzebehandlung, das zu Polymeren mit einer verbesserten Verarbeitbarkeit führt.

WO 2004/037903 A2 offenbart ein Verfahren zur Herstellung eines absorbierenden Polymergebildes durch Behandeln des Aussenbereiches eines unbehandelten absorbierenden Polymergebildes, umfassend die Schritte: in Kontakt bringen des Aussenbereiches des unbehandelten, absorbierenden Polymergebildes mit einer wässrigen Lösung enthaltend mindestens einen chemischen Vernetzer und mindestens eine anorganische Verbindung in kolloiddisperser Form; Erhitzen des absorbierenden Polymergebildes, dessen Aussenbereich mit der wässrigen Lösung in Kontakt gebracht wurde auf eine Temperatur im Bereich von 40 bis 300°C, so dass der Aussenbereich des absorbierenden Polymergebildes im Vergleich zum Innenbereich stärker vernetzt ist und die anorganische Verbindung im Aussenbereich des absorbierenden Polymergebildes mindestens teilweise immobilisiert wird. Das in Kontakt bringen eines absorbierenden, unbehandelten Polymergebildes mit einer Mischung aus pyrogener Kieselsäure, Fällungskieselsäure oder Kieselsäuresol und einem Salz umfassend ein $Al^{3+}$-Ion wird allerdings in WO 2004/037903 A2 nicht beschrieben.

Im Beispiel 6 der DE-A-199 09 653 wurde Pulver F, ein wasserabsorbierendes Polymergebilde, mit Ethylencarbonat (1,3-Dioxolan-2-on) und Aluminiumsulfat-18-Hydrat vermischt und 30 min bei 180°C erhitzt. Das unbehandelte Pulver F hatte eine Retention von 38 g/g ("TB") und eine SFC von 0, wobei durch die Behandlung die SFC auf 45 anstieg, die Retention allerdings auf 31 g/g abfiel. Das erhaltene Pulver hatte eine AAP 0.7 von 24,5 g/g und kann zB Babywindeln oder Damenbinden eingebracht werden. Ein Verbund ist ebenfalls offenbart.

In WO 2006/111404 A2 wird ein Verfahren zur Behandlung der Oberfläche wasserabsorbierender Polymergebilde, umfassend die Verfahrensschritte i) Bereitstellen eines unbehandelten, wasserabsorbierenden Polymergebildes; ii) in Kontakt bringen der Oberfläche des unbehandelten, wasserabsorbierenden Polymergebildes mit einer Kombination aus einem Metallsalz und einem Oxid eines Metalls bei einer Temperatur in einem Bereich von 100 bis 300 °C vorgestellt. Keine Erwähnung findet dabei das in Kontakt bringen eines absorbierenden, unbehandelten Polymergebildes mit einer Mischung aus pyrogener Kieselsäure, Fällungskieselsäure oder Kieselsäuresol und einem Salz, umfassend ein $Al^{3+}$-Ion.

Der Nachteil der aus dem Stand der Technik bekannten wasserabsorbierenden Polymergebilde besteht jedoch unter anderem darin, dass absorbierende Strukturen, wie etwa absorbierende Kerne in Windeln herkömmliche Polymergebilde mit hoher Aufnahmekapazität als Absorptionsmitteln beinhalten, die bei einem plötzlichen Eintritt von besonders großen Körperflüssigkeitsmengen, wie er etwa bei erwachsenen Windelträgern oder aber beim Einnässen einer Windeln durch ein an *Enuresis nocturna* ("Bettnässen") leidendem Kind in fortgeschrittenem Alter auftreten kann, nicht in der Lage sind, die Flüssigkeitsmenge insbesondere unter einer durch den liegenden Windelträger verursachten Druckbelastung vollständig aufzunehmen und im Inneren der absorbierenden Struktur weiter zu verteilen.

Allgemein liegt der Erfindung die Aufgabe zugrunde, die sich aus dem Stand der Technik ergebenden Nachteile zu überwinden.

Insbesondere lag der vorliegenden Erfindung die Aufgabe zugrunde, wasserabsorbierende Polymergebilde mit hoher Aufnahmekapazität zur Verfügung zu stellen, welche im Vergleich zu herkömmlichen Absorptionsmitteln bei einem Einsatz in absorbierenden Strukturen, wie zum Beispiel Windeln, eine verbesserte Flüssigkeitsaufnahme und Weiterverteilung ermöglichen. Insbesondere sollen die wasserabsorbierenden Polymergebilden mit hoher Aufnahmekapazität in der Lage sein, auch bei einem Einsatz in Windeln für Erwachsene oder für an *Enuresis nocturna* erkrankten Windelträgern eine schnelle Aufnahme und gleichmäßige Weiterverteilung von plötzlich eintretenden, besonders großen Körperflüssigkeitsmengen unter einer Druckbelastung sicherzustellen.

Zudem liegt eine andere erfindungsgemäße Aufgabe darin, ein Verfahren anzugeben, mit dem solche wasserabsorbierenden Polymergebilde in einfacher, möglichst kontinuierlicher Weise und mit möglichst geringen Mengen organischer Lösungsmittel darstellbar sind. Bei diesem Herstellungsverfahren sollten sich zugesetzte pulverförmige Hilfsstoffe allenfalls in geringen Mengen von den Polymergebilden ablösen und die Polymereigenschaften nicht nachteilig beeinflussen.

Einen Beitrag zur Lösung der vorstehend genannten Aufgaben leistet ein Verfahren zur Herstellung oberflächennachvernetzter wasserabsorbierender Polymergebilde umfassend die Verfahrensschritte:

I. Bereitstellen eines unbehandelten, wasserabsorbierenden Polymergebildes (Pu) mit einer gemäß der hierin beschriebenen Testmethode bestimmten Retention von mindestens 37,5 g/g, vorzugsweise von mindestens 38 g/g, besonders bevorzugt mindestens 39 g/g, darüber hinaus bevorzugt mindestens 41 g/g und am meisten bevorzugt mindestens 43 g/g, wobei vorzugsweise ein Retentionswert von 75 g/g, besonders bevorzugt 70 g/g. darüber hinaus bevorzugt 65 g/g, darüber hinaus noch mehr bevorzugt 60 g/g und am meisten bevorzugt 55 g/g nicht überschritten wird;

II. in Kontakt bringen dieses unbehandelten, wasserabsorbierenden Polymergebildes (Pu) mit einer Mischung aus

(a) pyrogener Kieselsäure, Fällungskieselsäure oder Kieselsäuresol und
(b) einem Salz umfassend ein $Al^{3+}$-Ion,

wobei die wasserabsorbierenden Polymergebilde während oder nach dem Verfahrensschritt II. oberflächennachvernetzt werden.

Unter einem "unbehandelten, wasserabsorbierenden Polymergebilde (Pu)" wird dabei ein Polymergebilde verstanden, welches noch nicht mit der Mischung aus (a) und (b) in Kontakt gebracht worden ist.

In einer Ausgestaltung des erfindungsgemäßen Verfahrens ist es bevorzugt, dass die Retention des unbehandelten, wasserabsorbierenden Polymergebildes in einem Bereich zwischen 40 und 50 g/g liegt.

Vollkommen überraschend, dafür aber nicht minder vorteilhaft, wurde festgestellt, dass durch die Oberflächenbehandlung von Precurser-Partikeln mit einer hohen Retention von mindestens 37,5 g/g, welche üblicherweise durch eine vernachlässigbare Permeabilität gekennzeichnet sind, wasserabsorbierende Polymergebilde erhalten werden können, welche in absorbierenden Strukturen mit hohem Superabsorberanteil ein sehr gutes Flüssigkeitsaufnahme- und Flüssigkeitsverteilungsverhalten zeigen.

Erfindungsgemäß bevorzugte wasserabsorbierende Polymergebilde sind Fasern, Schäume oder Teilchen, wobei Fasern und Teilchen bevorzugt und Teilchen besonders bevorzugt sind. Wasserabsorbierende Polymergebilde in diesen Formen werden erhalten, in dem als unbehandelte, wasserabsorbierende Polymergebilde (Pu) in entsprechender Weise Fasern, Schäume oder Teilchen eingesetzt werden.

Erfindungsgemäß bevorzugte wasserabsorbierende Polymerfasern sind so dimensioniert, dass sie in oder als Garne für Textilien und auch direkt in Textilien eingearbeitet werden können. Es ist erfindungsgemäß bevorzugt, dass die wasserabsorbierenden Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß besonders bevorzugte wasserabsorbierende Polymerteilchen sind so dimensioniert, dass sie eine mittlere Teilchengröße gemäß ERT 420.2-02 im Bereich von 10 bis 3000 $\mu$m, vorzugsweise 20 bis 2000 $\mu$m und besonders bevorzugt 150 bis 850 $\mu$m oder 150 bis 600 $\mu$m aufweisen. Weiterhin ist es erfindungsgemäß bevorzugt, dass die erfindungsgemäßen wasserabsorbierenden Polymerteilchen zu mindestens 30 Gew.-%, vorzugsweise zu mindestens 40 Gew.-% und am meisten bevorzugt zu mindestens 50 Gew.-% auf Partikeln mit einer Partikelgröße in einem Bereich von 300 bis 600 $\mu$m basieren.

Das im Verfahrensschritt I. des erfindungsgemäßen Verfahren bereitgestellte unbehandelte, wasserabsorbierende Polymergebilden (Pu) ist bevorzugt ein Polymergebilde, welches auf

($\alpha$1) 20-99,999 Gew.-%, bevorzugt 55 bis 98,99 Gew.-% und besonders bevorzugt 70 bis 98, 79 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,

($\alpha$2) 0-80 Gew.-%, vorzugsweise 0-44,99 Gew.-% und besonders bevorzugt 0,1-44,89 Gew.-% polymerisierten, monoethylenisch ungesättigten, mit ($\alpha$1) copolymerisierbaren Monomeren,

($\alpha$3) 0,001 - 5 Gew.-%, vorzugsweise 0,01 - 3 Gew.-% und besonders bevorzugt 0,01- 0,5 Gew.-% eines oder mehrerer Vernetzer,

($\alpha$4) 0-30 Gew.-%, vorzugsweise 0-5 Gew.-% und besonders bevorzugt 0,1-5 Gew.-% eines wasserlöslichen Polymeren,

($\alpha$5) 0 bis 20 Gew.-%, vorzugsweise 2,5 bis 15 Gew.-% und besonders bevorzugt 3 bis 6 Gew.-% Wasser, sowie

($\alpha$6) 0-20 Gew.-%, vorzugsweise 0 bis 10 Gew.-% und besonders bevorzugt 0,1-8 Gew.-% eines oder mehrerer Hilfsmittel basiert, wobei die Summe der Gewichtsmengen ($\alpha$1) bis ($\alpha$6) 100 Gew.-% beträgt.

Die monoethylenisch ungesättigten, säuregruppenhaltigen Monomere ($\alpha$1) können teilweise oder vollständig, bevorzugt teilweise neutralisiert sein. Vorzugsweise sind die monoethylenisch ungesättigten, säuregruppenhaltigen Monomere zu mindestens 23mol-%, besonders bevorzugt zu mindestens 50 Mol-% und darüber hinaus bevorzugt zu 50-80 Mol-% neutralisiert In diesem Zusammenhang wird auf DE 195 29 348 A1 und deren Offenbarung verwiesen. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed-Bed Ion-Exchange Absorbent Polymers"* (MBIEA-Polymere) bezeichnet und sind unter anderem in der WO-A-99/34843 offenbart. Auf die Offenbarung der WO-A-99/34843 wird hiermit verwiesen. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Fomaldacin, Melamin und dergleichen, sowie deren sekundäre oder tertiäre Aminderivate.

Als ethylenisch ungesättigte, säuregruppenhaltige Monomere ($\alpha$1) werden vorzugsweise diejenigen Verbin-

dungen eingesetzt, die in der WO-A-2004/037903, auf die hiermit verwiesen wird, als ethylenisch ungesättigte, säuregruppenhaltige Monomere (α1) genannt werden. Besonders bevorzugte ethylenisch ungesättigte, säuregruppenhaltige Monomere (α1) sind Acrylsäure, und Methacrylsäure, wobei Acrylsäure am meisten bevorzugt ist.

Gemäß einer Ausführungsform des erfindungsgemäßen Verfahrens werden als unbehandelte, wasserabsorbierende Polymergebilden (Pu) Polymergebilde eingesetzt, bei denen die monoethylenisch ungesättigten, mit (α1) copolymerisierbaren Monomere (α2) Acrylamide, Methacrylamid oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformanamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform des erfindungsgemäßen Verfahrens werden als unbehandelte, wasserabsorbierende Polymergebilde (Pu) Polymergebilde eingesetzt, bei denen die monoethylenisch ungesättigten, mit (α1) copolymerisierbaren Monomere (α2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit (α1) copolymerisierbare Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat bevorzugt.

Die monoethylenisch ungesättigten, mit (α1) copolymerisierbaren Monomere (α2) umfassend weiterhin Methylpolyethylenglykol-allylether, Vinylacetat, Styrol und Isobutylen.

Als Vernetzer (α3) werden vorzugsweise diejenigen Verbindungen eingesetzt, die in der WO-A-2004/037903 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat.

Als wasserlösliche Polymere (α4) können in den wasserabsorbicrenden, unbehandelten Polymergebilden (Pu) wasserlösliche Polymerisate, wie teil- oder vollverseifter Polyvinylalkohol, Polyvinylpyrrolidon, Stärke oder Stärkederivate, Polyglykole oder Polyacrylsäure enthalten sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α6) können in den wasserabsorbierenden, unbehandelten Polymergebilden (Pu) Stellmittel, Geruchsbinder, oberflächenaktive Mittel oder

Antioxidatien enthalten sein. Weiterhin sind in den wasserabsorbierenden Polymerteilchen (Pu) als Hilfsstoffe vorzugsweise diejenigen von den zu polymerisierenden Monomeren (α1) und (α2), den Vernetzern (α3) und den gegebenenfalls vorhandenen wasserlöslichen Polymeren (α4) verschiedenen Komponenten enthalten, die zur radikalischen Polymerisation eingesetzt werden. Zu diesen Komponenten gehören insbesondere die Initiatoren sowie gegebenenfalls Kettenregler.

In einer besonderen Ausführungsform des erfindungsgemäßen Verfahrens werden als wasserabsorbierende, unbehandelte Polymergebilde (Pu) Polymergebilde eingesetzt, die zu mindestens 50 Gew.-%, vorzugsweise zu mindestens 70 Gew.-% und darüber hinaus bevorzugt zu mindestens 90 Gew.-% auf carboxylatgruppenhaltigen Monomeren basieren. Es ist erfindungsgemäß weiterhin bevorzugt, dass die Komponente (α1) zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Aus den vorgenannten Monomeren und Vernetzern lässt sich das wasserabsorbierende, unbehandelte Polymergebilde (Pu) durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen. Bevorzugt wird die Lösungspolymerisation in Wasser als Lösungsmittel durchgeführt.

Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Bei der inversen Suspensions- oder Emulsionspolymerisation wird eine wässrige, teilneutralisierte Acrylsäurelösung mit Hilfe von Schutzkolloiden oder Emulgatoren in einem hydrophoben, organischen Lösungsmittel dispergiert und durch Radikalinitiatoren die Polymerisation gestartet. Nach Beendigung der Polymerisation wird das Wasser aus dem Reaktionsgemisch azeotrop entfernt und das Polymerprodukt abfiltriert und getrocknet. Die Vernetzungsreaktion kann durch Einpolymerisieren eines polyfunktionellen Vernetzers, der in der Monomerenlösung gelöst ist, und/oder durch Reaktion geeigneter Vernetzungsmittel mit funktionellen Gruppen des Polymeren während einer der Herstellungsschritte erfolgen. Das Verfahrensprinzip ist z. B. in US 4,340,706, DE 37 13 601 und DE 28 40 010 beschrieben.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO-A-2004/037903 als mögliche Initiatoren genannt werden.

Besonders bevorzugt wird zur Herstellung der wasserabsorbierenden Polymergebilde (Pu) ein Redoxsystem bestehend aus Wasserstoffperoxid, Natriumperoxodisulfat und Ascorbinsäure eingesetzt.

Die Trocknung des nach der Polymerisation erhaltenen Hydrogels erfolgt vorzugsweise bei Temperaturen, die üblicherweise im Bereich von 80 bis 200°C liegen.

Die Trocknung erfolgt vorzugsweise in dem Fachmann bekannten Öfen oder Trocknern, beispielsweise in Bandtrocknern, Hordentrocknern, Drehrohröfen, Wirbelbetttrocknern, Tellertrocknern, Paddeltrocknern oder Infrarottrocknern. Sollten die so erhaltenen, getrockneten Polymere noch nicht in partikulärer Form vorliegen, so müssen sie nach der Trocknung noch zerkleinert werden. Das Zerkleinern erfolgt dabei vorzugsweise durch Trockenmahlen, vorzugsweise durch Trockenmahlen in einer Hammermühle, einer Stiftmühle, einer Kugelmühle oder einer Walzenmühle. Nach dem Zerkleinern ist es weiterhin bevorzugt, dass die Polymergebilde auf eine durch Siebanalyse bestimmte Partikelgröße von bis zu 1.000 $\mu$m und besonders bevorzugt von bis zu 850 $\mu$m abgesiebt werden, wobei das Gewichtsmittel der Partikelgröße vorzugsweise in einem Bereich von 150 bis 850 $\mu$m, besonders bevorzugt zwischen 200 bis 600 $\mu$m liegt.

Es ist erfindungsgemäß besonders bevorzugt, dass das im Verfahrensschritt I. eingesetzte unbehandelte, wasserabsorbierende Polymergebilde (Pu) ein vernetztes Polyacrylat in partikulärer Form ist, welches durch Polymerisation einer Acrylsäure in Gegenwart eines der in der WO-A-2004/037903 genannten Vernetzers in wässriger Lösung, beinhaltend die Acrylsäure in einer Menge in einem Bereich von 5 bis 80 Gew.-%, vorzugsweise 10 bis 70 Gew.-% und besonders bevorzugt 20 bis 50 Gew.-%, bezogen auf das Gewicht der wässrigen Lösung, und anschließendes Zerkleinern des erhaltenen Hydrogels, Trocknen des zerkleinerten Hydrogels auf einen Wassergehalt in einem Bereich von 1 bis 50 Gew.-%, vorzugsweise 2,5 bis 40 Gew.-% und besonders bevorzugt 5 bis 30 Gew.-%, und gegebenenfalls weiteres Zermahlen des getrockneten Hydrogels erhalten wurde.

Weiterhin ist es bevorzugt, dass die im Verfahrensschritt II. eingesetzten unbehandelten, wasserabsorbierenden Polymergebilde (Pu), sofern es sich bei den Polymergebilden um Teilchen handelt, zu mindestens 30 Gew.-%, vorzugsweise zu mindestens 40 Gew.-% und am meisten bevorzugt zu mindestens 50 Gew.-% auf Partikeln mit einer Partikelgröße in einem Bereich von 300 bis 600 $\mu$m basieren.

Im Verfahrensschritt II. des erfindungsgemäßen Verfahrens werden die unbehandelten, wasserabsorbierenden Polymergebilde (Pu) mit einem permeabilitätssteigernden Mittel in Kontakt gebracht, wobei das permeabilitätssteigernde Mittel vorzugsweise in einer Menge von mindestens 0,001 Gew.-%, besonders bevorzugt im Bereich von 0,1 bis 10 Gew.-% und darüber hinaus bevorzugt im Bereich von 0,5 bis 5 Gew.-%, bezogen auf das Gewicht des unbehandelten, wasserabsorbierenden Polymergebildes (Pu), mit dem Polymergebilde (Pu) in Kontakt gebracht wird. Bei diesem permeabilitätssteigernden Mittel handelt es sich um eine Mischung aus

(a) pyrogener Kieselsäure, Fällungskieselsäure oder Kieselsäuresol und
(b) einem Salz umfassend ein Al$^{3+}$-Ion.

Kieselsäuresole werden in der DE 102 49 821, auf deren Offenbarung hinsichtlich der Kieselsäuresole hingewiesen wird, beschriebene. Die kommerziell erhältlichen, unter der Bezeichnung Aerosil® bekannten pyrogenen Kieselsäuren, welche vorzugsweise ein Teilchengröße im Bereich von 5 bis 50 nm, besonders bevorzugt im Bereich von 8 bis 20 nm aufweisen, sind bevorzugt. Weitere bevorzugte SiO-Verbindungen sind Fällungskieselsäuren, wie sie beispielsweise unter der Bezeichnung Sipernat® kommerziell erhältlich sind.

Bevorzugt ist pyrogene Kieselsäure, wie sie beispielsweise unter der Handelbezeichnung Aerosil® erhältlich ist, Fällungskieselsäuren, wie sie kommerziell unter der Bezeichnung Sipernat® erhältlich sind, oder Kieselsäuresol, wie es beispielsweise unter der Handelsbezeichnung Levasil® erhältlich ist

Vorzugsweise wird die Komponente (a) der Mischung in einer Menge in einem Bereich von mindestens 0,001 Gew.-%, besonders bevorzugt von 0,1 bis 10 Gew.-% und darüber hinaus bevorzugt von 0,5 bis 5 Gew.-%, bezogen auf das unbehandelte, wasserabsorbierende Polymergebilde (Pu), mit dem unbehandelten, wasserabsorbierenden Polymergebilde (Pu) in Kontakt gebracht.

Als Salze umfassend ein mehrwertiges, vorzugsweise dreiwertiges Kation werden Salze eingesetzt, welche $Al^{3+}$-Ionen umfassen. Unter diesen Salzen besonders bevorzugt sind Salze beinhaltend Chlorid-Anionen, Iodid-Anionen, Bromid-Anionen, Nitrat-Anionen, Nitrit-Anionen, Sulfid-Anionen, Sulfit-Anionen, Sulfat-Anionen, Carbonat-Anionen, Hydrogencarbonat-Anionen, Hydroxid-Anionen, Acetat-Anionen oder Oxalat-Anionen. Bevorzugte Salze sind Aluminiumchlorid, Polyaluminiumchlorid, Aluminiumsulfat, Aluminiumnitrat, bis-Aluminium-Kalium-Sulfat, bis-Aluminium-Natrium-Sulfat Alumi-niumlactat, Aluminiumoxalat, Aluminiumcitrat, Aluminiumglyoxylat, Aluminiumsuccinat, Aluminiumitaconat, Aluminiumerotonat, Aluminiumbutyrat, Aluminiumsorbat, Alumi-niummalonat, Aluminiumbenzoat, Aluminiumtartrat, Aluminiumpyruvat, Alumiumvalerat, Alummiumformat, Aluminiumglutarat, Aluminiumpropanat und Aluminiumacetat, wobei $AlCl_3 \times 6H_2O$, $NaAl(SO_4)_2 \times 12\ H_2O$, $Al(NO_3)_3 \times 9\ H_2O$, $KAl(SO_4)_2 \times 12\ H_2O$ oder $Al_2(SO_4)_3 \times 14\text{-}18\ H_2O$ sowie die entsprechenden wasserfreien Salze, am meisten bevorzugt sind. Eine weitere bevorzugte Verbindung, die als Salz aufgefasst wird, ist $Al(O)OH$.

Hinsichtlich des Salzes umfassend ein $Al^{3+}$-Ion ist es des weiteren bevorzugt, dass das Salz in einer Menge in einem Bereich von mindestens 0,001 Gew.-%, besonders bevorzugt in einer Menge in einem Bereich von 0,1 bis 10 Gew.-% und darüber hinaus bevorzugt in einer Menge in einem Bereich von 0,5 bis 5 Gew.-%, jeweils bezogen auf das Gewicht des unbehandelten, wasserabsorbierenden Polymergebilde (Pu), mit dem unbehandelten, wasserabsorbierenden Polymergebilde (Pu) in Kontakt gebracht wird.

Das unbehandelte, wasserabsorbierende Polymergebilde (Pu) wird sowohl mit der Komponenten (a) der Mischung als auch mit dem Salz umfassend ein $Al^{3+}$-Ion in Kontakt gebracht, dabei kann

i) das unbehandelte, wasserabsorbierende Polymergebilde (Pu) zeitgleich mit den beiden Komponenten in Kontakt gebracht werden, in dem beispielsweise beide Komponenten zunächst unter trockenen Bedingungen vermischt und dann diese Mischung mit dem unbehandelten, wasserabsorbierenden Polymergebilde (Pu) in Kontakt gebracht wird, oder indem beide Komponenten in Form eines gemeinsamen Fluids mit dem unbehandelten, wasserabsorbierenden Polymergebilde (Pu) in Kontakt gebracht werden.

Das in Kontakt bringen des unbehandelten, wasserabsorbierenden Polymergebildes (Pu) mit der Mischung aus (a) und (b) unter trockenen Bedingungen oder in Form eines Fluids erfolgt vorzugsweise in dem Fachmann bekannten Mischaggregaten wie z. B. dem Patterson-Kelley-Mischer, dem DRAIS-Turbulenzmischer, dem Lödigemischer, dem Ruberg-Mischer, dem Schneckenmischer, dem Tellermischer und dem Wirbelschichtmischer sowie in kontinuierlich arbeitenden senkrechten Mischern, in denen das Polymergebilde mittels rotierender Messer in schneller Frequenz gemischt wird (Schugi-Mischer).

Es ist erfindungsgemäß weiterhin bevorzugt, dass, sofern nicht während des Verfahrensschrittes II. eine Oberflächennachvernetzung durchgeführt wird, das in Kontakt bringen des unbehandelten, wasserabsorbierenden Polymergebildes (Pu) mit der Mischung aus (a) und (b) bei Temperaturen in einem Bereich von 10 bis 100°C, besonders bevorzugt in einem Bereich von 15 bis 60°C und darüber hinaus bevorzugt bei 20 bis 40°C erfolgt, wobei ein in Kontakt bringen bei Raumtemperatur am meisten bevorzugt ist.

Des weiteren sind die durch das erfindungsgemäße Verfahren erhältlichen wasserabsorbierenden Polymergebilde oberflächennachvernetzt. Die Oberflächennachvernetzung erfolgt dabei während oder nach dem Verfahrensschritt II., wobei zur Nachvernetzung vorzugsweise chemische Nachvernetzer eingesetzt werden.

Unter chemischen Nachvernetzern werden dabei vorzugsweise Verbindungen verstanden, die mindestens zwei funktionelle Gruppen aufweisen, die mit funktionellen Gruppen eines Polymers in einer Kondensationsreaktion (=Kondensationsvernetzer), in einer Additionsreaktion oder in einer Ringöffnungsreaktion reagieren können.

Als Nachvernetzer besonders bevorzugt sind Polyole, beispielsweise Ethylenglykol, Polyethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriamin, Triethylentetraamin, Tetraethylenpentaamin oder Pentaethylenhexaamin, Polyglycidylether-Verbindungen wie Ethylenglykoldiglycidylether, Polyethylenglykoldiglycidylether, Glycerindiglycidylether, Glycerinpolyglycidylether, Pentareritritpolyglycidylether, Propylenglykoldiglycidylether, Polypropylenglykoldiglycidylether, Neopentylglykoldiglycidylether, Hexandiolglycidylether, Trimethylolpropanpolyglycidylether, Sorbitolpolyglycidylether, Phthalsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbulanol-tris[3-(1-azi-ridinyl)propionat], 1,6-Hexamethylendiethylenharnstoff und Diphenylmethan-bis-4,4'-N,N'-diethylenharnstoff, Halogenepoxide beispielsweise Epichlor- und Epibromhydrin und $\alpha$-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Nachvernetzer sind des weiteren Polyoxazoline wie 1,2-Ethylenbisoxazolin, Nachvernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Unter den vorstehend genannten Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on oder Poly-1,3-dioxolan-2-on, wobei Ethylencarbonat als Nachvernetzer am meisten bevorzugt ist.

Der Nachvernetzer wird im erfindungsgemäßen Verfahren vorzugsweise in einer Menge im Bereich von 0,01 bis 30 Gew.-%, besonders bevorzugt 0,1, bis 20 Gew.-% und darüber hinaus bevorzugt von 0,3 bis 5 Gew.-%, bezogen auf das wasserabsorbierender Polymergebilde, eingesetzt.

Insbesondere dann, wenn der Nachvernetzer unter den Druck- und Temperaturbedingungen der Nachvernetzung nicht flüssig ist, wird der Nachvernetzer in Form eines Fluids $F_3$ umfassend den Nachvernetzer und ein Lösungsmittel eingesetzt, wobei als Lösungsmittel vorzugsweise Wasser, mit Wasser mischbare organische Lösungsmittel wie etwa Methanol, Ethanol, 1-Propanol, 2-Propanol oder 1-Butanol oder aber Mischungen aus Wasser und diesen organischen Lösungsmitteln eingesetzt werden. Dabei ist der Nachvernetzer, sofern er zusammen mit einem Lösungsmittel eingesetzt wird, in dem Fluid $F_3$ vorzugsweise in einer Menge in einem Bereich von 5 bis 75 Gew.-%, besonders bevorzugt in einer Menge in einem Bereich von 20 bis 40 Gew.% und am meisten bevorzugt in einer Menge in einem Bereich von 5 bis 25 Gew.-%, jeweils bezogen auf das Gesamtgewicht des Fluids $F_3$, in dem Fluid $F_3$ enthalten.

Das in Kontakt bringen des Fluids $F_3$ mit dem wasserabsorbierenden Polymergebilde erfolgt vorzugsweise in den im Zusammenhang mit dem in Kontakt bringen des unbehandelten, wasserabsorbierenden Polymergebildes (Pu) mit der Komponente (a) der Mischung bzw. mit dem Salz umfassend ein $Al^{3+}$-Ion genannten Mischaggregaten.

Nachdem der Nachvernetzer bzw. das Fluid $F_3$ mit den wasserabsorbierenden Polymergebilden in Kontakt gebracht wurde, erfolgt die Nachvernetzungsreaktion durch Erhitzen des wasserabsorbierenden Polymergebildes auf Temperaturen im Bereich von 40 bis 300°C, bevorzugt von 80 bis 275°C und besonders bevorzugt von 125 bis 250°C. Die optimale Zeitdauer der Nachvernetzung kann für die einzelnen Nachvernetzertypen leicht ermittelt werden. Sie wird dadurch begrenzt, wenn das gewünschte Eigenschaftsprofil des wasserabsorbierenden Polymergebildes infolge Hitzeschädigung wieder zerstört wird. Die thermische Behandlung kann in üblichen Trocknern oder Öfen durchgeführt werden, beispielhaft seien Drehrohröfen, Wirbelbetttrockner, Tellertrockner, Paddeltrockner oder Infrarottrockner genannt

Wenn die Nachvernetzung während des Verfahrensschrittes II. erfolgt, so ist es bevorzugt, den Nachvernetzer zusammen mit der Mischung aus (a) und (b) mit dem unbehandelten, wasserabsorbierenden Polymergebilde (Pu) in Kontakt zu bringen. Sofern das in Kontakt bringen der Polymergebilde mit der Mischung aus (a) und (b) nicht unter trockenen Bedingungen erfolgt, so kann der Nachvernetzer auch in einem Fluid $F_1$ oder dem Fluid $F_2$ gelöst oder dispergiert werden. Nach dem in Kontakt bringen der unbehandelten, wasserabsorbierenden Polymergebilde (Pu) mit dem Fluid $F_1$ bzw. $F_2$ erfolgt dann die Nachvernetzung des Polymergebildes durch das Erhitzen auf die vorstehend genannten Temperaturen.

Wenn die Nachvernetzung nach dem Verfahrensschritt II. erfolgt, so werden die unbehandelten, wasserabsorbierenden Polymergebilde (Pu) zunächst in der vorstehend beschriebenen Art und Weise mit der Mischung aus (a) und (b) in Kontakt gebracht. Anschließend erfolgt die Oberflächennachvernetzung durch in Kontakt bringen der Polymergebilde mit dem Nachvernetzer und anschließendes Erhitzen.

Gemäß einer besonderen Ausführungsform des erfindungsgemäßen Verfahrens zur Herstellung wasserabsorbierender Polymergebilde wird im Verfahrensschritt ii) ein unbehandeltes, jedoch vorzugsweise bereits oberflächennachvernetztes wasserabsorbierendes Polymergebildes mit der Mischung gemäß Anspruch 1 in Kontakt gebracht, wobei die Mischung in Pulverform vorliegt.

Bei dieser besonderen Ausführungsform des erfindungsgemäßen Verfahrens zur Herstellung wasserabsorbierender Polymergebilde umfasst das Verfahren vorzugsweise die folgenden Verfahrensschritte:

i) Bereitstellen des unbehandelten, vorzugsweise jedoch bereits oberflächennachvernetzten wasserabsorbierenden Polymergebildes;

ii) in Kontakt bringen des unbehandelten, vorzugsweise jedoch bereits oberflächennachvernetzten wasserabsorbierenden Polymergebildes mit einer Feinstteilchenkomponente umfassend eine Mischung aus (a) und (b), vorzugsweise bei einer Temperatur in einem Bereich von 30 bis 300°C, besonders bevorzugt von 100 bis 300°C, darüber hinaus bevorzugt in einem Bereich von 125 bis 250°C und am meisten bevorzugt in einem Bereich von 150 bis 200°C.

In diesem Zusammenhang ist es insbesondere bevorzugt, dass mindestens 50 Gew.-%, besonders bevorzugt mindestens 75 Gew.%, darüber hinaus noch mehr bevorzugt mindestens 95 Gew.% und am meisten bevorzugt mindestens 99 Gew.-% des in Pulverform vorliegenden Salzes umfassend ein $Al^{3+}$-Ion einen mittleren Teilchendurchmesser (Gewichtsmittel) in einem Bereich von 10 bis 1.000 $\mu m$, vorzugsweise von 50 $\mu m$ bis 800 $\mu m$, besonders bevorzugt von 100 bis 600 $\mu m$ und am meisten bevorzugt von 200 bis 400 $\mu m$ aufweisen, jeweils bestimmt durch dem Fachmann bekannte Verfahren zur Partikelgrößenbestimmung, beispielsweise durch Siebanalyse oder mittels eines Coulter-Counters. Weiterhin ist es bevorzugt, dass mindestens 50 Gew.-%, vorzugsweise mindestens 75 Gew.-% und am meisten bevorzugt mindestens 90 Gew.-% der SiO-Verbindung eine durch Siebanalyse (für Partikelgrößen größer als 10 $\mu m$) oder Laserdiffraktometrie (für Partikelgrößen kleiner als 10 $\mu m$) bestimmte Partikelgröße in einem Bereich von 10 bis 1.000.000 nm, besonders bevorzugt in einem Bereich von 12 bis 500.000 nm und am meisten bevorzugt in einem Bereich von 15 bis 5000 nm aufweisen. Weiterhin ist es bevorzugt, dass die SiO-Verbindung ein Gewichtsmittel der Partikelgröße in einem Bereich von 15 bis 5000 nm, vorzugsweise in einem Bereich von 20 bis 3000 nm und am meisten bevorzugt in einem Bereich von 100 bis 2000 nm aufweist.

Weiterhin kann es bei dieser besonderen Ausführungsform des erfindungsgemäßen Verfahrens bevorzugt sein, wenn die Feinstteilchenkomponente neben der pulverförmigen Mischung aus (a) und (b) zusätzlich einen Binder beinhaltet, wobei auch dieser Binder vorzugsweise in partikulärer Form vorliegt und insbesondere zu mindestens 50 Gew.%, besonders bevorzugt zu mindestens 75 Gew.-%, darüber hinaus noch mehr bevorzugt zu mindestens 95 Gew.% und am meisten bevorzugt zu mindestens 99 Gew.-% auf Partikel mit einem mittleren Teilchendurchmesser (Gewichtsmittel) in einem Bereich von 10 bis 1.000 $\mu m$, vorzugsweise von 50 $\mu m$ bis 800 $\mu m$, besonders bevorzugt von 100 bis 600 $\mu m$ und am meisten bevorzugt von 200 bis 400 $\mu m$ basiert, jeweils bestimmt durch dem Fachmann bekannte Verfahren zur Partikelgrößenbestimmung, beispielsweise durch Siebanalyse oder mittels eines Coulter-Counters.

In diesem Zusammenhang ist es insbesondere bevorzugt, dass der Binder als eine Binderhauptkomponente eine organische Verbindung beinhaltet, wobei die organische Verbindung vorzugsweise bei 20°C ein Feststoff ist.

Besonders bevorzugt ist es, dass die organische Verbindung ein vorzugsweise lineares Polymer ist, vorzugsweise ein lineares Polymer ausgewählt aus der Gruppe umfassend Polyurethane, Polyester, Polyamide, Polyesteramide, Polyolefine, Polyvinylester, Polyether, Polystyrole, Polyimide, insbesondere Polyetherimide, Polyimine, Schwefelpolymere, insbesondere Polysulfon, Polyacetale, insbesondere Polyoxymethylene, Fluorkunststoffe, insbesondere Polyvinylidenfluorid, Styrol-Olefin-Copolymere, Polyacrylate, Ethylen-Vinylacetat-Copolymere oder Gemische aus zwei oder mehr der genannten Polymere, wobei unter diesen Polymeren Polykondensate und unter diesen Polyether besonders bevorzugt und lineare Polyether am meisten bevorzugt sind.

Besonders geeignete lineare Polyether umfassen Polyalkylenglykole, insbesondere Polyethylenglykole, Polypropylenglykole, Poly(ethylen/propylen)glykole mit statistischer oder blockartiger Anordnung der Ethylen- oder Propylen-Monomere oder Mischungen aus mindesten zwei dieser Polyalkylenglykole.

Weitere geeignete, vorzugsweise lineare Polymere sind diejenigen Polymere, die in der DE-A-103 34 286 als "thermoplastische Klebstoffe" genannt werden. Auf den Offenbarungsgehalt der DE-A-103 34 286 hinsichtlich thermoplastischer Klebstoffe wird hiermit verwiesen.

Wenn neben der Mischung aus (a) und (b) auch ein Binder eingesetzt wird, ist es insbesondere bevorzugt, dass das in Kontakt bringen der Oberfläche des unbehandelten, vorzugsweise bereits oberflächennachvernetzten wasserabsorbierenden Polymergebildes mit der Feinstteilchenkomponente bei einer Temperatur in einem Bereich von 30 bis 200°C, besonders bevorzugt von 50 bis 160°C und am meisten bevorzugt in einem Bereich von 70 bis 140°C erfolgt. Bei diesen Temperaturen kommt es insbesondere auch zu einer Immobilisierung der Feinstteilchen auf der Oberfläche des unbehandelten, wasserabsorbierenden Polymergebildes.

Die Menge an Bindemittel, sofern eingesetzt, liegt vorzugsweise in einem Bereich von 0,0001 bis 5 Gew.-% und besonders bevorzugt in einem Bereich von 0,001 bis 2 Gew.-%, jeweils bezogen auf das Gewicht der wasserabsorbierenden Polymergebilde. Das Gewichtsverhältnis zwischen Feinstteilchenkomponente und Bindemittel liegt vorzugsweise in einem Bereich von Feinstteilchenkomponente: Bindemittel von 20 : 1 bis 1 : 20, besonders bevorzugt von 10 : 1 bis 1 : 10 und am meisten bevorzugt von 10 : 1 bis 2 : 1.

Bei der vorstehend beschriebenen, besonderen Ausführungsform des erfindungsgemäßen Verfahrens, bei der eine pulverförmige Mischung aus (a) und (b) eingesetzt wird, umfasst das Verfahren neben der Bereitstellung des unbehandelten, vorzugsweise bereits oberflächennachvernetzten wasserabsorbierenden Polymergebildes im Verfahrensschritt i) auch die Bereitstellung einer Feinstteilchenkomponente umfassend die pulverförmige Mischung aus (a) und (b) sowie den pulverförmigem Binder. Hinsichtlich der Art und Weise des in Kontakt bringens der Feinstteilchenkomponente mit dem unbehandelten, wasserabsorbierenden Polymergebilde sind unterschiedliche Verfahrensführungen denkbar:

- Gemäß der Variante $V_A$ wird zunächst im Verfahrensschritt ii) eine Mischung aus Feinstteilchenkomponente und unbehandelten, vorzugsweise jedoch bereits oberflächennachvernetzten wasserabsorbierenden Polymergebilden hergestellt und diese anschließend Zwecks Immobilisierung der Feinstteilchen auf die vorstehend genannte Temperatur erhitzt, wobei das wasserabsorbierende Polymergebilde zwar schon mit dem Nachvernetzter in Kontakt gebracht worden ist, aber noch nicht auf eine für eine Oberflächennachvernetzung erforderliche Temperatur erhitzt worden ist

- Gemäß der Variante $V_B$ werden zunächst vor dem Verfahrensschritt ii) die unbehandelten, vorzugsweise jedoch bereits oberflächennachvernetzten wasserabsorbierenden Polymergebilde auf die vorstehend beschriebene Temperatur erhitzt und anschließend im Verfahrensschritt ii) diese vorgewärmten wasserabsorbierenden Polymergebilde mit der nicht-vorgewärmten Feinstteilchenkomponente vermischt.

- Gemäß der Variante $V_C$ werden zunächst vor dem Verfahrensschritt ii) die unbehandelten, wasserabsorbierenden Polymergebilde und die Feinstteilchenkomponente getrennt jeweils auf die vorstehend beschriebene Temperatur erwärmt und anschließend im Verfahrensschritt ii) die vorgewärmten wasserabsorbierenden Polymergebilde mit der ebenfalls vorgewärmten Feinstteilchenkomponente vermischt. Gemäß einer besonderen Ausgestaltung dieser Variante $V_C$ ist es bevorzugt, die Feinstteilchenkomponente nach dem Erwärmen und vor dem Vermischen mit den vorgewärmten wasserabsorbierenden Polymergebilden zunächst abzukühlen, vorzugsweise auf eine Temperatur in einem Bereich von 10 bis 100°C, besonders bevorzugt 15 bis 75°C und am meisten bevorzugt 20 bis 60°C, danach gegebenenfalls zu zerkleinern, beispielsweise mittels eines Mörsers, und dann die abgekühlte und gegebenenfalls zerkleinerte Feinstteilchenkomponente mit den vorgewärmten wasserabsorbierenden Polymergebilden zu vermischen.

- Gemäß der Variante $V_D$ wird zunächst vor dem Verfahrensschritt ii) die Feinstteilchenkomponente auf die vorstehend beschriebene Temperatur erwärmt und anschließend im Verfahrensschritt ii) die vorgewärmte Feinstteilchenkomponente mit den nicht-vorgewärmten, unbehandelten, wasserabsorbierenden Polymergebilde vermischt. Gemäß einer besonderen Ausgestaltung dieser Variante $V_D$ ist es bevorzugt, die Feinstteilchenkomponente nach dem Erwärmen und vor dem Vermischen mit den nicht-vorgewärmten wasserabsorbierenden Polymergebilden zunächst abzukühlen, vorzugsweise auf eine Temperatur in einem Bereich von 10 bis 100°C, besonders bevorzugt 15 bis 75°C und am meisten bevorzugt 20 bis 60°C, danach gegebenenfalls zu zerkleinern, beispielsweise mittels eines Mörsers, und dann die abgekühlte und gegebenenfalls zerkleinerte Feinstteilchenkomponente mit den nicht-vorgewärmten wasserabsorbierenden Polymergebilden zu vermischen.

Weiterhin kann es im Zusammenhang mit der vorstehend beschriebenen, besonderen Ausführungsform des erfindungsgemäßen Verfahrens, bei der eine pulverförmige Mischung aus (a) und (b) eingesetzt wird, vorteilhaft sein, wenn sich an den Verfahrensschritt ii) noch ein weiterer Verfahrensschritt iii) anschließt, in dem die Mischung aus unbehandeltem, wasserabsorbierenden Polymergebilde und Feinstteilchenkomponente noch für eine Zeitraum in einem Bereich von 10 Minuten bis 5 Stunden, besonders bevorzugt von 30 Minuten bis 3 Stunden gemischt wird, um eine möglichst homogene Verteilung der Feinstteilchen und der absorbierenden Polymergebilde zu ermöglichen, wobei hierzu dem Fachmann bekannte Mischvorrichtungen eingesetzt werden können. In diesem weiteren Verfahrensschritt kann die Mischung aus unbehandeltem, wasserabsorbierenden Polymergebilde und Feinstteilchenkomponente mit der Temperatur, die sie nach dem Immobilisieren im Verfahrensschritt ii) aufweist, in den Mischer eingebracht werden, wobei die Mischung dann im Verlaufe des Mischens vorzugsweise stetig auf eine niedrigere Temperatur, vorzugsweise auf Raumtemperatur, abgekühlt, werden kann.

Es handelt sich bei den erfindungsgemäß erhältlichen wasserabsorbierenden Polymergebilden vorzugsweise um Fasern, Schäume oder Teilchen, wobei Fasern und Teilchen bevorzugt und Teilchen besonders bevorzugt sind. Dabei weisen die Fasern bzw. die Teilchen die eingangs im Zusammenhang mit dem erfindungsgemäßen Verfahren zur Herstellung wasserabsorbierender Polymere genannten Fasermaße bzw. Teilchengrößenverteilung auf.

Weiterhin sind die durch das erfindungsgemäße Verfahren erhältlichen wasserabsorbierenden Polymergebilde dadurch gekennzeichnet, dass auf der Oberfläche der Polymergebilde ein permeabilitässteigerndes Mittel vorzugsweise in einer Menge von mindestens 0,001 Gew.%, besonders bevorzugt im Bereich von 0,1 bis 10 Gew.-% und darüber hinaus bevorzugt im Bereich von 0,5 bis 5 Gew.-% immobilisiert ist. Bei diesem permeabilitätssteigernden Mittel handelt es sich um - eine Mischung aus (a) und (b) gemäß Anspruch 1.

Gemäß einer besonderen Ausführungsform handelt es sich bei den durch das erfindungsgemäße Verfahren erhältlichen wasserabsorbierenden Polymergebilde um Polymerteilchen, welche eine durch Siebanalyse bestimmte Partikelgröße in einem Bereich von 10 bis 3000 $\mu$m, vorzugsweise 20 bis 2000 $\mu$m und besonders bevorzugt 150 bis 850 $\mu$m aufweisen, wobei die Polymerteilchen zu mindestens 50 Gew.-%, vorzugsweise zu mindestens 70 Gew.-% und darüber hinaus bevorzugt zu mindestens 90 Gew.-% auf carboxylatgruppenhaltigen Monomeren, vorzugsweise auf Acrylsäure, basieren, wobei diese carboxylatgruppenhaltigen Monomere vorzugsweise zu mindestens 20 Mol-%, besonders bevorzugt zu mindestens 50 Mol-% und darüber hinaus bevorzugt in einem Bereich von 60 bis 85 Mol-% neutralisiert sind. Weiterhin ist es bevorzugt, dass die erfindungsgemäß erhältlichen wasserabsorbierenden Polymergebilde, sofern es sich bei den Polymergebilden um Teilchen handelt, zu mindestens 30 Gew.%, vorzugsweise zu mindestens 40 Gew.-% und am meisten bevorzugt zu mindestens 50 Gew.-% auf Partikeln mit einer Partikelgröße in einem Bereich von 300 bis 600 mm basieren.

Gemäß einer besonderen Ausführungsform der erfindungsgemäß erhältlichen wasserabsorbierenden Polymergebilde sind diese des weiteren dadurch gekennzeichnet, dass ein gemäß der hierin beschriebenen Testmethode hergestellter Airlaid-Verbund bestehend aus 350 g/m$^2$ Zellulosefaser, 18 g/m$^2$ einer Bikomponentenfaser, 350 g/m$^2$ der erfindungsgemäßen wasserabsorbierende Polymergebilde und 36 g/m$^2$ Tissue durch mindestens eine der folgenden Eigenschaften gekennzeichnet ist:

($\gamma$1) ein gemäß der hierin beschriebenen Testmethode bestimmter Rewet-Wert nach dem dritten Einnässen von maximal 3 g, bevorzugt von maximal 2,9 g und besonders bevorzugt von maximal 2,8 g;

($\gamma$2) eine gemäß der hierin beschriebenen Testmethode bestimmte Acquisition Time nach dem zweiten Einnässen von maximal 450 s, bevorzugt von maximal 400 s und besonders bevorzugt von maximal 380 s;

($\gamma$3) eine gemäß der hierin beschriebenen Testmethode bestimmte Acquisition Time nach dem dritten Einnässen von maximal 1500 s, bevorzugt von maximal 1400 s und besonders bevorzugt von maximal 1300 s.

Gemäß einer besonderen Ausführungsform der erfindungsgemäß erhältlichen wasserabsorbierenden Polymergebilde ist ein gemäß der hierin beschriebenen Testmethode hergestellter Airlaid-Verbund durch folgende Eigenschaften oder Eigenschaftskombinationen gekennzeichnet: ($\gamma$1), ($\gamma$2), ($\gamma$3), ($\gamma$1)($\gamma$2), ($\gamma$1)($\gamma$3), ($\gamma$2)($\gamma$3), ($\gamma$1)($\gamma$2)($\gamma$3).

Einen weiteren Beitrag zur Lösung der eingangs genannten Aufgaben leistet ein Verbund beinhaltend die durch das erfindungsgemäße Verfahren erhältliche oberflächennachvernetzte wasserabsorbierende Polymergebilde und ein Substrat. Vorzugsweise sind die erfindungsgemäßen wasserabsorbierenden Polymergebilde und das Substrat fest miteinander verbunden. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt.

Erfindungsgemäß handelt es sich bei dem Verbund um eine Sauglage.

Die Sauglage weist vorteilhafterweise mindestens einer der folgenden Eigenschaften auf:

($\delta$1) einen gemäß der hierin beschriebenen Testmethode bestimmter Rewet-Wert nach dem dritten Einnässen von maximal 3 g, bevorzugt von maximal 2,9 g und besonders bevorzugt von maximal 2,8 g;

($\delta$2) eine gemäß der hierin beschriebenen Testmethode bestimmte Acquisition Time nach dem zweiten Einnässen von maximal 450 s, bevorzugt von maximal 400 s und besonders bevorzugt von maximal 380 s;

($\delta$3) eine gemäß der hierin-beschriebenen Testmethode bestimmte Acquisition Time nach dem dritten Einnässen von maximal 1500 s, bevorzugt von maximal 1490 s und besonders bevorzugt von maximal 1480 s.

Gemäß einer besonderen Ausführungsform der erfindungsgemäßen Sauglage ist diese durch folgende Eigenschaften oder Eigenschaftskombinationen gekennzeichnet: ($\delta$1), ($\delta$2), ($\delta$3), ($\delta$1)($\delta$2), ($\delta$1)($\delta$3), ($\delta$2)($\delta$3), ($\delta$1)($\delta$2)($\delta$3).

Erfindungsgemäß besonders bevorzugten Sauglagen sind diejenigen Sauglagen, die in der US 5,599,335 als "absorbent members" beschrieben werden. Es wird auf den Offenbarungsgehalt der US 5,599,335 verwiesen, insbesondere hinsichtlich der in den Sauglagen enthaltenen Fasern und Hilfsstoffe sowie hinsichtlich der Verfahren zur Herstellung der Sauglagen.

Erfindungsgemäß umfasst dieser Verbund bzw. diese Sauglage mindestens einen Bereich, welcher das wasserabsorbierende Polymergebilde in einer Menge im Bereich von 15 bis 100 Gew.-%, vorzugsweise 30 bis 100 Gew.-%, besonders bevorzugt von 50 bis 99,99 Gew.%, ferner bevorzugt von 60 bis 99,99 Gew.% und darüber hinaus bevorzugt von 70 bis 99 Gew.-%, jeweils bezogen auf das Gesamtgewicht des betreffenden Bereichs des Verbundes bzw. der Sauglage, beinhaltet, wobei dieser Bereich vorzugsweise eine Größe von mindestens 0,01 cm$^3$, vorzugsweise mindestens 0,1 cm$^3$ und am meisten bevorzugt mindestens 0,5 cm$^3$ aufweist.

Weiterhin ist die erfindungsgemäße Sauglage vorzugsweise durch ein Flächengewicht von mindestens 0,02 g/cm$^2$, vorzugsweise von mindestens 0,03 g/cm$^2$, besonders bevorzugt im Bereich von 0,02 bis 0,12 g/cm$^2$ und darüber hinaus bevorzugt im Bereich von 0,03 bis 0,11, g/cm$^2$ gekennzeichnet, wobei die Sauglage des weiteren eine Dicke von maximal 20 mm, vorzugsweise maximal 15 mm und am meisten bevorzugt von maximal 10 mm aufweist.

Gemäß einer bevorzugten Ausführungsform der erfindungsgemäßen Sauglage weist diese eine Fläche von

maximal 500 cm$^2$, vorzugsweise maximal 350 cm$^2$, besonders bevorzugt maximal 300 cm$^2$ auf.

Die Herstellung des erfindungsgemäßen Verbundes erfolgt dadurch, dass die durch das erfindungsgemäße Verfahren erhältlichen wasserabsorbierenden Polymergebilde und das Substrat und ggf. das Hilfsmittel miteinander in Kontakt gebracht werden. Das in Kontakt bringen erfolgt vorzugsweise durch Wetlaid- und Airlaid-Verfahren, Kompaktieren, Extrudieren und Mischen.

Gemäß einer besonderen Ausgestaltung des Verfahrens zur Herstellung eines Verbundes umfasst dieses Verfahren die folgenden Verfahrensschritte:

A) Bereitstellen eines Substrates;

B) Bereitstellen eines unbehandelten, vorzugsweise jedoch bereits oberflächennachvernetzten, wasserabsorbierenden Polymergebildes;

C) Bereitstellen einer Feinstteilchenkomponente;

D) In Kontakt bringen des Substrates mit dem wasserabsorbierenden Polymergebilde;

E) in Kontakt bringen des wasserabsorbierenden Polymergebildes mit der Feinstteilchenkomponente;

F) Immobilisieren mindestens eines Teils der Feinstteilchen auf der Oberfläche der wasserabsorbierenden Polymergebilde.

Als Feinstteilchenkomponente ist dabei diejenige Feinstteilchenkomponente bevorzugt, die bereits vorstehend im Zusammenhang mit derjenigen besonderen Ausführungsform des erfindungsgemäßen Verfahren zur Herstellung wasserabsorbierenden Polymergebildes, bei der eine pulverförmige Mischung gemäß Anspruch 1 eingesetzt wird, als bevorzugte Feinstteilchenkomponente beschrieben worden ist, umfassend eine Mischung aus (a) und (b). Besonders bevorzugt ist eine pulverförmige Mischung dieser beiden Komponenten und pulverförmigem Binder.

Gemäß einer Variante dieser besonderen Ausgestaltung des Verfahrens zur Herstellung eines Verbundes werden zunächst das Substrat und das unbehandelte, vorzugsweise jedoch bereits oberflächennachvernetzte, wasserabsorbierende Polymergebilde miteinander in Kontakt gebracht, vorzugsweise dadurch, dass zunächst das Substrat vorgelegt und anschließend das Polymergebilde entweder gleichmäßig oder aber auf bestimmte Areale der Substratoberfläche aufgebracht, vorzugsweise gestreut wird. Anschließend werden dann die auf der Substratoberfläche befindlichen wasserabsorbierenden Polymergebilde mit der Feinstteilchenkomponente in Kontakt gebracht, beispielsweise dadurch, dass die Feinstteilchenkomponente auf die auf der Substratoberflächen befindlichen Polymergebilde gestreut wird. Schließlich erfolgt die Immobilisierung der Feinstteilchenkomponente auf der Oberfläche der Polymergebilde, wobei diese Immobilisierung vorzugsweise durch das vorstehend im Zusammenhang mit dem erfindungsgemäßen Verfahren zur Behandlung der Oberfläche wasserabsorbierender Polymergebilde beschriebene Erhitzen erfolgt. Bei dieser Variante der besonderen Ausgestaltung des Verfahrens zur Herstellung eines Verbundes erfolgt mithin der Verfahrensschritt E) nach dem Verfahrensschritt D).

Gemäß einer anderen Variante dieser besonderen Ausgestaltung des Verfahrens zur Herstellung eines Verbundes wird zunächst das Substrat vorgelegt Anschließend wird das unbehandelte, vorzugsweise jedoch bereits oberflächennachvernetzte, wasserabsorbierende Polymergebilde mit dem Substrat in Kontakt gebracht, vorzugsweise dadurch, dass zunächst das Substrat vorgelegt und anschließend das Polymergebilde entweder gleichmäßig oder aber auf bestimmte Areale der Substratoberfläche aufgebracht, vorzugsweise gestreut wird. Noch bevor das Polymergebilde mit der Substratoberfläche in Kontakt gebracht wird, werden die wasserabsorbierenden Polymergebilde mit der Feinstteilchenkomponente in Kontakt gebracht, beispielsweise dadurch, dass die Feinstteilchenkomponente mit dem Polymergebilde, bevor es auf die Substratoberfläche gestreut wird, vermischt wird. Nach dem die Polymergebilde mit dem Substrat in Kontakt gebracht wurden, erfolgt dann die Immobilisierung der Feinstteilchenkomponente auf der Oberfläche der Polymergebilde. Bei dieser Variante der besonderen Ausgestaltung des Verfahrens zur Herstellung eines Verbundes erfolgt mithin der Verfahrensschritt E) vor dem Verfahrensschritt D).

Dichtungsmaterialien, flüssigkeitsaufnehmende Hygieneartikel, Träger für pflanzen- oder pilzwachstumsregulierende Mittel oder Pflanzenschutzwirkstoffe, Zusätze für Baustoffe, Verpackungsmaterialien oder Bodenzusätze.

Außerdem betrifft die Erfindung die Verwendung der erfindungsgemäßen wasserabsorbierenden Polymergebilde, der durch das erfindungsgemäße Verfahren erhältlichen wasserabsorbierenden Polymergebilde, des Verbundes oder des durch das vorstehend beschriebene Verfahren erhältlichen Verbundes in den vorstehend genannten chemischen Produkten, insbesondere in Hygieneprodukten, zur Hochwasserbekämpfung, zur Isolierung gegen Wasser, zur Regulierung des Wasserhaushalts von Böden oder zur Behandlung von Lebensmitteln.

Die Erfindung wird nun anhand von Figuren, Testmethoden und nicht limitierenden Beispielen näher erläutert.

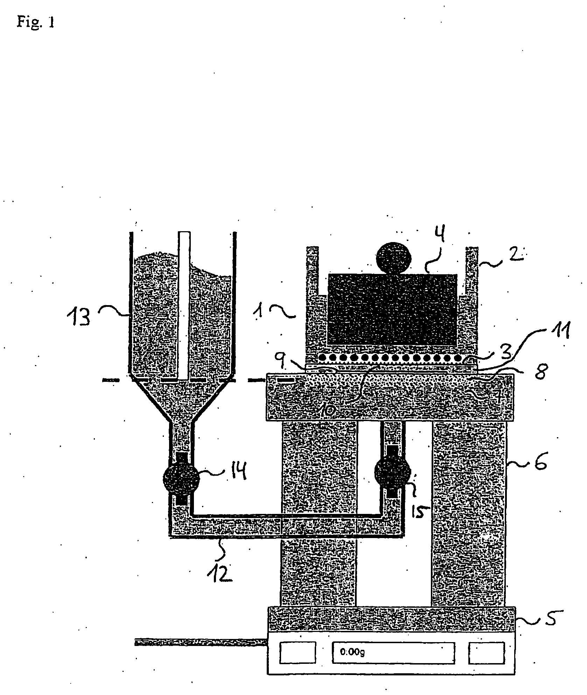

Fig. 1: die Figur 1 zeigt die Vorrichtung zur Bestimmung des TAAP-Wertes mittels des hierin beschriebenen Testverfahrens.

**TESTMETHODE**

BESTIMMUNG DES SFC-WERTES

Die Bestimmung der Permeabilität im gequollene Zustand (*Saline Flow Conductivity* = SFC) erfolgt nach einer in WO-A-95/22356 beschriebenen Methode. In einem Zylinder mit Siebboden werden ca. 0,9 g Superabsorbermaterial (bei Partikeln die gesamte Partikelfraktion) eingewogen und sorgfältig auf der Siebfläche verteilt Das Superabsorbermaterial lässt man in JAYCO synthetischem Urin 1 Stunde lang gegen einen Druck von 20 g/cm² quellen. Nach Erfassung der Quellhöhe des Superabsorbers lässt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Messtemperatur 20-25°C) während der Messung bezüglich der Gelbett-Beschaffenheit gewährleistet. Der auf den gequollenen Superabsorber wirkende Druck ist weiterhin 20 g/cm². Mit Hilfe eines Computers und einer Waage wird die Flüssigkeitsmenge, die die Gelschicht als Funktion der Zeit passiert, in Intervallen von 20 Sekunden innerhalb einer Zeitperiode von 10 Minuten erfasst. Die Fliessrate g/s durch die gequollene Gelschicht wird mittels Regressionsanalyse mit Extrapolation der Steigung und Ermittlung des Mittelpunktes auf den Zeitpunkt t=0 der Fließmenge innerhalb der Minuten 2-10 ermittelt. Der SFC-Wert (K) wurde in cm³·s·g⁻¹ angegeben und wie folgt berechnet:

$$K = \frac{F_s(t=0) \cdot L_0}{r \cdot A \cdot \Delta P_1} = \frac{F_s(t=0) \cdot L_0}{139506}$$

wobei

$F_s(t=0)$     die Fließrate in g/s,
$L_0$     die Dicke der Gelschicht in cm,
r     die Dichte der NaCl-Lösung (1,003 g/cm³),
A     die Fläche der Oberseite der Gelschicht im Messzylinder (28,27 cm²),
$\Delta P$     der hydrostatische Druck, der auf der Gelschicht lastet (4.920 dyne/cm²), und
K     der SFC-Wert ist.

BESTIMMUNG PER RETENTION

Zur Bestimmung der Retention des wasserabsorbierenden Polymermaterials (bei Partikeln die gesamte Partikelfraktion) wird der sogenannte Teebeuteltest durchgeführt. Als Prüflösung wurde eine 0.9 Gew.-%ige NaCl-Lösung verwendet

1 g wasserabsorbierendes Polymermaterial werden eingewogen($W_1$) und in einen Teebeutel eingeschweißt. Der Teebeutel wird für 30 Minuten in die Prüflösung gelegt und anschließend in einer Schleuder (23 cm Durchmesser, 1.400 UpM) 3 Minuten geschleudert und erneut gewogen ($W_2$). Einen Teebeutel ohne wasserabsorbierendes Polymermaterial, dessen Gewicht nach dem Schleudern ebenfalls bestimmt wird ($W_3$), lässt man als Blindwert mitlaufen. Die Retention wurde in g/g angegeben und wie folgt berechnet:

$$\text{Retention [g/g]} = \frac{W_2 - W_3 - W_1}{W_1}$$

BESTIMMUNG DER TIME-DEPENDENT ABSORPTION AGAINST PRESSURE (TAAP)

Das zu testende absorbierende Material wird in eine Apparatur 1 bestehend aus einen Plexiglaszylinder 2, welcher einen Innendurchmesser von 60 mm, eine Höhe von 50 mm und als Boden 3 ein Siebgewebe aus Stahl (400 mesh) aufweist, auf den Boden 3 gestreut und mit einem definierten Gewicht 4 belastet (bei dem Gewicht 4 handelt es sich um einen in den Plexiglaszylinder 2 passgenau einführbaren zylinderförmigen Stempel, in den seinerseits ein Metallgewicht eingeführt werden kann). Das Gesamtgewicht des Gewichtes 4 ist so zu wählen, dass auf das absorbierende Material ein Druck von 20 g/cm$^2$ lastet. Die Apparatur 1 wird auf ein auf einer Waage 5 befindliches Gestell 6, welches eine zylinderförmige Vertiefung 7 für eine Glasfritte 8 mit einem Durchmesser von 70 mm (porosity = 0 from Schott-Keramikfilter Duran, Germany) aufweist, gestellt, wobei zwischen der Glasfritte 8 und dem Siebboden 3 des Plexiglaszylinders 2 ein Filterpapier 9 (Schleicher & Schuell, Schwarzband 589/1; Durchmesser 45 mm), zentrisch auf dem Filterpapier 9 eine weitere Glasfritte 10 mit einem Durchmesser von 20 mm und auf der Glasfritte 8 ein Distanzring 11 mit einem Innendurchmesser von 60 mm, einer Dicke von 5 mm und einer Höhe, welche genau der Höhe der Glasfritte 10 entspricht, gelegt wird. Die Glasfritte 8 ist über einen Silikonschlauch 12 fluidleitend mit einem Flüssigkeitsreservoir 13 (bei dem es sich um einen 500ml- Tropftrichter mit Glasrohr handelt) verbunden, im dem sich 0,9 Gew.-%ige NaCl-Lösung, befindet. Weiterhin kann über die Ventile 14 und 15 der Transport der NaCl-Lösung vom Flüssigkeitsreservoir 13 zur Vertiefung 7 und somit zur Glassfritte 8 wahlweise geöffnet oder geschlossen werden.

Zunächst werden, ohne dass sich die Apparatur 1 auf dem Gestell 6 und die Glasfritte 8 in der Vertiefung 7 des Gestells 6 befindet, die Ventile 14 und 15 geöffnet, wobei das Flüssigkeitsreservoir sich auf einer solchen Höhe befindet, dass die Vertiefung 7 bis zum Rand mit der Testflüssigkeit gefüllt ist. Anschließend wird die Glasfritte 8 in die Vertiefung 7 eingelegt und das Filterpapier 9 auf die Glasfritte 8 gelegt, wobei sichergestellt wird, dass sich Glasfritte 8 und Filterpapier 9 ausreichend mit der Flüssigkeit vollgesaugt haben. Überschüssige Testflüssigkeit wird mit einem saugendem Material aufgenommen. Die Glasfritte 10 wird mittig auf das Filterpapier 9 gelegt. Es ist darauf zu achten, das sich die Glasfritte 10 ebenfalls vollständig mit Tostflüssigkeit voll saugt.

Nun werden 1,8 g $\pm$ 0,005 g des zu testenden absorbierenden Materials auf den Siebboden 3 der Apparatur 1 gleichmäßig gestreut und die Probe wird mit dem Gewicht 4 belastet. Auf einer von der Waage 5 verschiedenen Waage (Messgenauigkeit 0,01 g) wird das Gewicht der Apparatur bestimmt ($m_1$). Anschließend wird die Apparatur 1 vor das Gestell 6 auf die Waage 5 gestellt. Die Waage 5 wird tariert.

Im Anschluss daran wird die Messung gestartet, in dem die Apparatur 1 auf Glasfritte 10 und Distanzring 11, welche auf der mit Filterpapier 9 belegten Glasfritte 8, welche sich in der Vertiefung 7 befindet, gestellt wird. In Intervallen von 10 Sekunden nach Aufstellen der Apparatur 1 wird die jeweilige Aufnahme an Testflüssigkeit, $m_{abs\ 0s}$ bis $m_{abs\ 3600s}$, durch das absorbierende Material aufgezeichnet. Dieses geschieht durch den Einsatz eines rechnergestützten Aufzeichnungsprogrammes (Weighing Inn 1.0, Herbert Werth 1999©, es kann aber auch beispielsweise ein "Visual Basic for Application"-Modul für Microsoft® Excel© verwendet werden). Die Messdauer beträgt eine Stunde. Erreicht die daraus resultierende Absorptionskurve nicht die maximal mögliche Absorption, dadurch charakterisiert, dass die Veränderung des Messwertes zum Ende der Messung > 0,1 g/60 s ist, so wird die Apparatur 1 auf eine Glasfritte gestellt, welche der Glasfritte 8 gleicht und welche in einer bis zum oberen Rand der Glasfritte mit Restflüssigkeit befüllten Petrischale mit einem Mindestdurchmesser von 150 mm liegt. Die Testflüssigkeitsaufnahme wird in Abständen von 1 bis 60 Minuten kontrolliert, bis die Aufnahme < 0,1 g/60 s ist. Die Apparatur 1 wird dann auf einer von Waage 5 verschiedenen Waage (Genauigkeit 0,01 g) gewogen ($m_2$). Die maximale Absorption errechnet sich aus:

$$\text{Abs.}_{max} = m_2 - m_1$$

Über die Gewichtsmessungen mittels der Waage 5 werden die absoluten Mengen an Testflüssigkeitsaufnahme zu den definierten Zeiten ausgezeichnet. In einem Absorptions$_{absolut}$/Zeit-Diagramm wird die zu einem gegebenen Zeitpunkt absorbierte Flüssigkeitsmenge $m_{abs\ Xs}$ gegen die Zeit t aufgetragen. Zusätzlich wird ein Absorptions$_{relativ}$ / Zeit-Diagramm erstellt. Hierzu wird jeder Messwert $m_{abs\ Xs}$ in Prozent, bezogen auf Abs.$_{max}$, gegen die Zeit t aufgetragen.

$$m_{abs\ Xs}\ [\%] = (m_{abs\ Xs}\ [g] * 100) / \text{Abs.}_{max}\ [g]$$

Aus dem Absorptions$_{relativ}$ / Zeit-Diagramm kann dann abgelesen werden, ob und nach welcher Zeit 50 % bzw. 90 % der maximalen Absorption (Abs.$_{max}$) erreicht worden sind.

BESTIMMUNG DER ACQUISITION TIME UND DES REWET

Die Bestimmung der Aquisition Time und des Rewet erfolgte gemäß den in der DE-A-102 49 822 beschriebenen Testverfahren.

Dabei wird zunächst ein Airlaid-Verbund auf der Basis der M & J Fibretch Technologie hergestellt. Eine gepresste Zellulosefaser (mit dem Flächengewicht $m_{(Fluff\ Pulp)}$ und der Breite $b_{(Fluff\ Pulp)}$, Hersteller: Stora Enso, Finnland; Typ: Stora EFsemi-treated) mit der Geschwindigkeit $v_{(Fluff\ Pulp-Hammermühle)}$ in eine Hammermühle eingefahren und zerfasert. Ein Gebläse saugt die Fasern kontinuierlich ab. In denselben Luftstrom wird eine Bikomponentenfaser (Herstelle: Danaklon, jetzt FiberVisions, Dänemark; Typ: AL-Thermal-C phil 6 mm, 3,3 dtex, 40 raw white) dosiert. Dazu wird die Menge $m_{(Bikomponentenfaser/Abwurf)}$ in eine automatische Wägeinrichtung eindosiert. Diese Vorrichtung entlädt die Bikomponentenmenge alle 40cm (Länge der Wägeinrichtung) auf ein kontinuierlich, mit der Geschwindigkeit $v_{(Bikomponentenfaserdosierung)}$, laufendes Förderband. Das wasserabsorbierende Polymergebilde wird mit einer gravimetrischen Dosierschnecke mit dem Massenfluss $m_{(wasserabsorbierendes Polymergebilde)}$ in eine Eintragsvorrichtung (Venturisystem) dosiert und in den Luftstrom auf der Druckseite des Gebläses eingebracht. Das Gemisch Faser/wasserabsorbierende Polymergebilde wird kontinuierlich auf das, mit einem Tissue (Flächengewicht $m_{(Tissue)}$; Hersteller; Finess Hygiene AB, Schweden; Typ: Art.-Nr. 50330, Qualität KB 1800, Diapor-tissue-open) belegte, Förderband der Airlaidanlage (Geschwindigkeit $v_{(Airlaid-Förderband)}$, Breite $b_{(Airlaid)}$) abgelegt. Vor dem Aufrollen am Ende des Förderbandes wird nochmals ein Tissue (Flächengewicht $m_{(Tissue)}$; Hersteller; Finess Hygiene AB, Schweden; Typ: Art.-Nr. 50330, Qualität KB 1800, Diapor-tissue-open) auf den Airlaid-Verbund gelegt und anschließend auf eine Papphülse aufgerollt. Der Airlaid-Verbund wird anschließend thermisch in einem kontinuierlich arbeitenden Umlufttrockner fixiert (Parameter: Durchlaufgeschwindigkeit $v_{(Trockner)}$, Temperatur $T_{(Trockner)}$, Luftgeschwindigkeit $v_{(Trockner Luft)}$, Verweilzeit $t_{(Trockner)}$.

Die einzelnen Parameter sind in der folgenden Tabelle noch einmal zusammengestellt:

| Parameter | Wert | Einheit |
|---|---|---|
| $b_{(Fluff Pulp)}$ | 140 | mm |
| $m_{(Fluff Pulp)}$ | 800 | $g/m^2$ |
| $v_{(Fluff Pulp-Hammermühle)}$ | 3,4 | m/min |
| $v_{(Airlaid-förderbend)}$ | $\cong 3,2$ | m/min |
| $b_{(Airlaid)}$ | $\cong 350$ | mm |
| $v_{(Bikomponentenfaserdosierung}$ ‡ | $\cong 0,8$ | m/min |
| $m_{(Bikomponentenfaser/Abwurf)}$ | 10 | g |
| $m_{(wasserabsorbierendes Polymergebilde)}$ | 380 | g/min |
| $m_{(Tissue)}$ (Top- und Bottomlayer) | je 18 | $g/m^2$ |
| $v_{(Trackner)}$ | $\cong 0,5$ | m/min |
| $T_{(Trockner)}$ | 165 | °C |
| $v_{(Trockner, Luft)}$ | 2,4 | m/min |
| $t_{(Trockner)}$ | $\cong 4$ | min |
| * Entspricht zwei Abwürfen pro Minute | | |

BEISPIELE

Herstellung eines unbehandelten wasserabsorbierenden Polymers

Eine Monomerlösung bestehend aus 1200 g Acrylsäure, 932,56 g 50%ige Natronlauge, 1 732,92g entiomsiertes Wasser, 1,8996 g Monoallylpolyethylenglykol-750-monoacrylsäureester, 0,6192 g Polyethylenglykol-300-diacrylat und 24 g Polyethylenglykol-750-methoxymonomethyacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (1,2 g Natriumperoxodisulfat m 38,8 g $H_2O$, 0,028 g 35,5%ige Wasserstoffperoxid-Lösung in 7,72 g $H_2O$ und 0,06 g Ascorbinsäure in 19,94 g $H_2O$) zugesetzt Nachdem die Endtemperatur von ca.103°C erreicht war, wurde das entstandene Gel zerkleinert und bei 150°C 120 Minuten lang getrocknet Das getrocknete Polymerisat wurde grob zerstoßen, gemahlen und auf ein Pulver mit einer Partikelgröße von 150 bis 850 $\mu$m gesiebt (=Pulver A). Dabei wurde dergestalt gemahlen und abgesiebt, dass das erhaltene Pulver A durch folgende Partikelgrößenverteilung gekennzeichnet war: Partikel zwischen 150 und 300 $\mu$m: 10 bis 20 Gew.-%; Partikel zwischen 300 und 600 $\mu$m: 40 bis 60 Gew.-%; Partikel zwischen 600 und 850 $\mu$m: 15 bis 40 Gew.%.

Das Pulver A weist eine Retention von 45 g/g und einen SFC-Wert von 0 s$\times$cm$^3$/g auf.

Oberflächennachvernetzung

100 g des Pulvers A wurde mit einer Lösung aus 1 g Ethylencarbonat und 2,5 g Wasser beschichtet und zur Durchführung der Nachvernetzung für 40 Minuten bei 170°C erhitzt. Es wurde ein Pulver B erhalten. Dieses Produkt zeigte 50 % der maximalen Absorption unter einem Druck von 20 g/cm$^2$ nach etwa 42 Minuten und 90 % der maximalen Absorption unter einem Druck von 20 g/cm$^2$ nach mehr als einer Stunde.

Beispiel 1 (nicht erfindungsgemäß)

100 g des Pulvers A wurde mit einer Lösung aus 1 g Ethylencarbonat, 2,5 g Wasser und 0,75 g Al$_2$(SO$_4$)$_3$ beschichtet und zur Durchführung der Nachvernetzung für 40 Minuten bei 170°C erhitzt. Es wurde ein Pulver C erhalten. Dieses Produkt zeigte 50 % der maximalen Absorption unter einem Druck von 20 g/cm$^2$ nach etwa 10 Minuten und 90 % der maximalen Absorption unter einem Druck von 20 g/cm$^2$ nach etwa 23 Minuten.

Beispiel 2 (erfindungsgemäß)

100 g des Pulvers A wurde mit einer Lösung aus 1 g Ethylencarbonat, 2,5 g Wasser und 1 g ZP 30 (Kieselsäuresol, Levasil® 200/30 der Firma Bayer) 0,75 g Al$_2$(SO$_4$)$_3$ beschichtet und zur Durchführung der Nachvernetzung für 40 Minuten bei 170°C erhitzt. Es wurde ein Pulver D erhalten. Dieses Produkt zeigte 50 % der maximalen Absorption unter einem Druck von 20 g/cm$^2$ nach etwa 13 Minuten und 90 % der maximalen Absorption unter einem Druck von 20 g/cm$^2$ nach etwa 27 Minuten.

Die wasserabsorbierenden Polymerpulver A bis D sind durch folgende Absorptionseigenschaften gekennzeichnet:

| Pulver | Retention[g/g] | AUL0,7$_{psi}$ [g/g] | SFC [10$^{-7}$ s$\times$cm$^3$/g] |
|---|---|---|---|
| A | 45,0 | nicht bestimmt | 0 |
| B | 38,8 | 16,5 | 0 |
| C | 37,2 | 15,0 | 0 |
| D | 33,3 | 19,5 | 0 |

Beispiel 3 (nicht erfindungsgemäß)

100 g des Pulvers A wurden im Trockenschrank auf 130°C vorgewärmt.

Eine Mischung aus 24 g Al$_2$(SO$_4$)$_3\times$14 H$_2$O, welches in einer Zentrifugalmühle gemahlen und auf eine Partikelgröße in einem Bereich von 300 bis 400 $\mu$m abgesiebt wurde, 30 g Nanox® 200 (Zinkoxidpulver der Firma Elementis Specialities, USA, mit einer BET-Oberflache von 17m$^2$/g und einer mittleren Partikelgröße von 60 nm) und 3,6 g Polyethylenglylcol 10.000 (Polyethylenglykol mit einem Molekulargewicht von 10.000 g/mol), welches ebenfalls in einer Zentrifugalmühle gemahlen und auf eine Partikelgröße von weniger als 300 $\mu$m abgesiebt wurde), wurde hergestellt. 1,15 g dieser Mischung wurden in einem Krups-Mixer unter Rühren mit dem vorgewärmten wasserabsorbierenden Polymergebilde vermischt.

Beispiel 4

Unter Einsatz der Pulver B, C und D (erfindungsgemäß) wurden gemäß den Angaben im Zusammenhang mit dem Testverfahren zur Bestimmung der Aquisition Time und des Rewets Airlaid-Verbunde bestehend aus 350 g/m$^2$ Zellulosefaser (Fluff), 18 g/m$^2$ einer Bikomponentenfaser, 350 g/m$^2$ der erfindungsgemäßen wasserabsorbierenden Polymergebilde und 36 g/m$^2$ Tissue hergestellt und die Aquisition Time sowie der Rewet bestimmt. Folgende Messwerte wurden erhalten:

| Pulver | 1st Aquisition Time [sec] | 2nd Aquisition Time [sec] | 3rd Aquisition Time [sec] | Rewet [g] |
|---|---|---|---|---|
| B | 38 | 806 | 2.477 | 3,2 |
| C | 22 | 378 | 1.472 | 2,6 |
| D | 24 | 307 | 1.053 | 2,6 |

Es ist zu sehen, dass das erfindungsgemäße Pulver D, obwohl es keine nachweisbar Permeabilität aufweist, ausgezeichnet in der Lage ist, in einem Verbund eintretende, große Flüssigkeitsmengen weiterzuverteilen (erkennbar an den vergleichsweise kleinen Werten für die Aquisition Time).

Bezugszeichenliste

| | |
|---|---|
| 1 | Apparatur zur Bestimmung der Absorption unter Druck |
| 2 | Plexiglaszylinder |
| 3 | Boden aus Siebgewebe |
| 4 | Gewicht |
| 5 | Waage |
| 6 | Gestell |
| 7 | Vertiefung |
| 8 | Glasfritte (Durchmesser 70 mm) |
| 9 | Filterpapier |
| 10 | Glasfritte (Durchmesser 20 mm) |
| 11 | Distanzring |
| 12 | Silikonschlauch |
| 13 | Flüssigkeitsreservoir mit Steigrohr |
| 14 | Ventil |
| 15 | Ventil |

**Patentansprüche**

1. Verfahren zur Herstellung oberflächennachvernetzter, wasserabsorbierender Polymergebilde umfassend die Verfahrensschritte:

   I. Bereitstellen eines unbehandelten, wasserabsorbierenden Polymergebildes (Pu) mit einer gemäss der hierin beschriebenen Testmethode bestimmten Retention von mindestens 37,5 g/g;
   II. in Kontakt bringen dieses wasserabsorbierenden, unbehandelten Polymergebildes (Pu) mit einer Mischung aus

      (a) Kieselsäuresol und
      (b) 0.1 bis 10 Gew.-% bezogen auf das Gewicht des unbehandelten, wasserabsorbierenden Polymergebildes (Pu) eines Salzes umfassend ein $Al^{3+}$-Ion,

   wobei die wasserabsorbierenden Polymergebilde während oder nach dem Verfahrensschritt II. oberflächennachvernetzt werden,
   wobei zur Nachvernetzung chemische Nachvernetzer eingesetzt werden,
   wobei unter chemischen Nachvernetzern Verbindungen verstanden werden, die mindestens zwei funktionelle Gruppen aufweisen, die mit funktionellen Gruppen eines Polymers in einer Kondensationsreaktion, in einer Additionsreaktion oder in einer Ringöffnungsreakion reagieren können.

2. Verfahren nach Anspruch 1, wobei das Salz ausgewählt ist aus der Gruppe bestehend aus $AlCl_3 * 6H_2O$, $NaAl(SO_4)_2 * 12\ H_2O$, $KAl(SO_4)_2 \times 12\ H_2O$ und $Al_2(SO_4)_3 \times 14\text{-}18\ H_2O$.

3. Verbund, beinhaltend ein oberflächennachvernetzes, wässerabsorbierendes Polymergebilde erhältlich durch ein Verfahren gemäss Anspruch 1 oder 2, ein Substrat und gegebenenfalls Hilfsmittel, wobei der Verbund eine Sauglage ist und wobei der Verbund mindestens einen Bereich umfasst, welcher das wasserabsorbierende Polymergebilde in einer Menge im Bereich von 15 bis 100 Gew.-%, bezogen auf das Gesamtgewicht des betreffenden Bereichs des Verbundes, beinhaltet.

4. Verbund nach Anspruch 3, wobei der Verbund ein Flächengewicht von mindestens 0,02 $g/cm^2$ aufweist.

5. Verbund nach einem der Ansprüche 3 bis 4, wobei der Verbund eine Dicke von maximal 20 mm aufweist.

## Claims

1. Process for producing surface-postcrosslinked water-absorbing polymer structures, comprising the process steps of

I. providing an untreated water-absorbing polymer structure (Pu) having a retention determined according to the test method described herein of at least 37.5 g/g;
II. contacting this water-absorbing untreated polymer structure (Pu) with a mixture of

(a) silica sol, and
(b) 0.1 to 10wt%, based on the weight of the untreated water-absorbing polymer structure (Pu), of a salt comprising an $Al^{3+}$ ion,

wherein the water-absorbing polymer structures are surface postcrosslinked during or after process step II,
wherein the postcrosslinking step utilizes chemical postcrosslinkers,
wherein chemical postcrosslinkers are to be understood as meaning compounds having two or more functional groups capable of reacting with functional groups of a polymer in a condensation reaction, in an addition reaction or in a ring-opening reaction.

2. Process according to Claim 1, wherein the salt is selected from the group consisting of $AlCl_3 * 6H_2O$, $NaAl(SO_4)_2 * 12H_2O$, $KAl(SO_4)_2 \times 12H_2O$ and $Al_2(SO_4)_3 \times 14\text{-}18\ H_2O$.

3. Composite comprising a surface-postcrosslinked water-absorbing polymer structure obtainable by a process according to Claim 1 or 2, a substrate and optionally auxiliaries, wherein the composite is an absorbent layer and wherein the composite includes at least one region comprising the water-absorbing polymer structure in an amount ranging from 15 to 100 wt%, based on the total weight of the relevant region of the composite.

4. Composite according to Claim 3, wherein the composite has a mass per unit area of at least 0.02 g/cm$^2$.

5. Composite according to either of Claims 3 and 4, wherein the composite has a thickness of not more than 20 mm.

## Revendications

1. Procédé de fabrication de structures polymères absorbant l'eau, post-réticulées en surface, comprenant les étapes de procédé suivantes :

I. la préparation d'une structure polymère absorbant l'eau non traitée (Pu) ayant une rétention déterminée selon la méthode d'essai décrite dans le présent document d'au moins 37,5 g/g ;
II. la mise en contact de cette structure polymère absorbant l'eau non traitée (Pu) avec un mélange de

(a) un sol de silice, et
(b) 0,1 à 10 % en poids, par rapport au poids de la structure polymère absorbant l'eau non traitée (Pu), d'un sel comprenant un ion $Al^{3+}$,

les structures polymères absorbant l'eau étant post-réticulées en surface pendant ou après l'étape de procédé II, des agents de post-réticulation chimiques étant utilisés pour la post-réticulation,
les agents de post-réticulation chimiques se rapportant à des composés qui comprennent au moins deux groupes fonctionnels pouvant réagir avec des groupes fonctionnels d'un polymère dans une réaction de condensation, dans une réaction d'addition ou dans une réaction d'ouverture de cycle.

2. Procédé selon la revendication 1, dans lequel le sel est choisi dans le groupe constitué par $AlCl_3 * 6\ H_2O$, $NaAl(SO_4)_2 * 12\ H_2O$, $KAl(SO_4)_2 \times 12\ H_2O$ et $Al_2(SO_4)_3 \times 14\text{-}18\ H_2O$.

3. Composite, contenant une structure polymère absorbant l'eau post-réticulée en surface pouvant être obtenue par un procédé selon la revendication 1 ou 2, un substrat et éventuellement un adjuvant, dans lequel le composite est une couche aspirante et dans lequel le composite comprend au moins une zone qui contient la structure polymère absorbant l'eau en une quantité dans la plage allant de 15 à 100 % en poids, par rapport au poids total de la zone en question du composite.

**4.** Composite selon la revendication 3, dans lequel le composite présente un poids superficiel d'au moins 0,02 g/cm$^2$.

**5.** Composite selon l'une quelconque des revendications 3 à 4, dans lequel le composite présente une épaisseur d'au plus 20 mm.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10016041 A
- WO 9848857 A
- WO 9849221 A
- WO 2004037903 A2
- DE 19909653 A
- WO 2006111404 A2
- DE 19529348 A1
- WO 9934843 A
- WO 2004037903 A
- US 4286082 A
- DE 2706135
- US 4076663 A
- DE 3503458

- DE 4020780
- DE 4244548
- DE 4323001
- DE 4333056
- DE 4418818
- US 4340706 A
- DE 3713601
- DE 2840010
- DE 10249821
- DE 10334286 A
- US 5599335 A
- WO 9522356 A
- DE 10249822 A